# EUROPEAN PATENT SPECIFICATION

(11) **EP 0 510 878 B1**
(45) Date of publication and mention of the grant of the patent: **23.12.1998**
(21) Application number: 92303457.3
(22) Date of filing: 16.04.1992
(51) Int. Cl.: C12N 15/12, C07K 14/705, G01N 33/58, G01N 33/68

(54) **Human neurokinin-1 receptor**
Menschlicher Neurokinin-1-Rezeptor
Récepteur humain de la neurokinine 1

(30) Priority: 25.04.1991 US 691197; 25.04.1991 US 691200; 25.04.1991 US 691198
(43) Date of publication of application: 28.10.1992
(73) Proprietor: Merck & Co., Inc., Rahway New Jersey 07065-0900 (US)
(72) Inventor: Fong, Tung Ming, Somerset, NJ 08873 (US); Strader, Catherine D., Verona, NJ 07044 (US)
(74) Representative: Thompson, John Dr.

(56) References cited:
- BIOCHEMICAL AND BIOPHYSICAL RESEARCH COMMUNICATIONS. vol. 180, no. 2, 31 October 1991, DULUTH, MINNESOTA US pages 1110 - 1117; Hopkins, B. et al.: 'Isolation and characterization of the human lung NK-1 receptor cDNA.'
- BIOCHEMICAL AND BIOPHYSICAL RESEARCH COMMUNICATIONS. vol. 179, no. 3, 30 September 1991, DULUTH, MINNESOTA US pages 1231 - 1240; Takeda Y;Chou KB;Takeda J;Sachais BS;Krause JE: 'Molecular cloning, structural characterization and functional expression of the human substance P receptor.'
- BIOCHEMISTRY. vol. 30, no. 44, 5 November 1991, EASTON, PA US pages 10640 - 10646; Gerard NP;Garraway LA;Eddy RL Jr;Shows TB;Iijima H;Paquet JL;Gerard C: 'Human substance P receptor (NK-1): organization of the gene, chromosome localization, and functional expression of cDNA clones.'
- SCIENCE. vol. 247, 23 February 1990, LANCASTER, PA US pages 958 - 952; Hershey AD;Krause JE: 'Molecular characterization of a functional cDNA encoding the rat substance P receptor.'
- JOURNAL OF BIOLOGICAL CHEMISTRY. vol. 264, no. 30, 25 October 1990, BALTIMORE US pages 17649 - 17652; Yokota Y;Sasai Y;Tanaka K;Fujiwara T;Tsuchida K;Shigemoto R;Kakizuka A;Ohkubo H;Nakanishi S: 'Molecular characterization of a functional cDNA for rat substance P receptor.'
- PATENT ABSTRACTS OF JAPAN vol. 015, no. 341 29 August 1991

## Description

### BACKGROUND OF THE INVENTION

The present invention concerns cloned human neurokinin-l receptor (human NKlR) and recombinant human NKlR. Neurokinin-1 receptor is also known as substance P receptor.

J. Yokota, et al., J. Biol. Chem., 264:17649 (1989) have reported cloned rat neurokinin-1 receptor. A.D. Hershey, et al., Science, 247:958 (1990) have reported the characterisation of cDNA encoding the rat neurokinin-1 receptor.

N.P. Gerard, et al., J. Biol. Chem., 265:20455 (1990), have reported human neurokinin-2 receptor. Cloned rat and bovine neurokinin-2 receptor have likewise been reported. See respectively, Y. Sasi, and S. Nakanishi, Biochem Biophys. Res. Comm., 165:695 (1989), and Y. Masu, et al., Nature 329:836 (1987). Cloned rat neurokinin-3 receptor has also been reported by R. Shigemoto, et al., J. Biol. Chem., 265:623 (1990).

The above references, however, neither disclose or suggest the instant invention. In particular, the pharmacological profile of the human receptor differs significantly from the rat. Moreover, the rat neurokinin-l receptor differs from the NKlR disclosed herein by 23 amino acids.

Substance P is a naturally occuring undecapeptide belonging to the tachykinin family of peptides. Substance P is a pharmacologically-active neuropeptide that is produced in mammals. Its characteristic amino acid sequence is illustrated in U.S. 4,680,283. As is well known in the art substance P and other tachykinins have been implicated in the pathophysiology of numerous diseases. Substance P has been shown to be involved in the transmission of pain or migraine (see B.E.B. Sandberg et al., Journal of Medicinal Chemistry, Vol. 25, p. 1009 (1982)), as well as in central nervous system disorders such as anxiety and schizophrenia, in respiratory and inflammatory diseases such as asthma and rheumatoid arthritis, respectively, and in gastrointestinal disorders and diseases of the GI tract, like ulcerative colitis and Crohn's disease, etc. (see D. Regoli in "Trends in Cluster Headache," edited by F. Sicuteri et al., Elsevier Scientific Publishers, Amsterdam, 1987, pp. 85-95).

The instant invention also concerns an assay protocol which can be used to determine substance P activity in body fluids. The assay can also be used for identifying and evaluating substances that bind substance P receptor. Thus, the assay can be used to identify substance P antagonists and evaluate their binding affinity. Other methods includes that described by M.A. Cascieri, et al., J. Biol. Chem., 258-5158 (1983).
By use of such methods, substance P antagonists have been identified. See, for example, R. M. Snider, et al., Science, 251:435 (Jan. 1991) and S. McLean, et al., Science, 251:437 (Jan. 1991). See also WO90/05525 which published May 31, 1990.

Methods to date have proven inferior, in part, for failure of the animal receptor (animal NKlR, NK2R or NK3R) activity to accurately reflect that of human neurokinin-1 receptor. Furthermore, prior to this disclosure human NKlR has not been available in a purified form or in substantial isolation from NK2R and/or NK3R.

Use of such neurokinin receptor sources can not accurately depict the affinity for human NK1R.

### BRIEF DESCRIPTION OF THE DRAWINGS

- Figure 1: Full length amino acid sequence of human neurokinin-l receptor.
- Figure 2: Full length nucleotide sequence of the cloned human neurokinin-l receptor complementary DNA.
- Figure 3: Competitive binding of substance P (SP), substance K (SK) and human neurokinin-1 receptor (NKlR) in COS assay.

### SUMMARY OF THE INVENTION

Recombinant human neurokinin-l receptor (human NKlR) is disclosed which has been prepared by polymerase chain reaction techniques. Also disclosed is the complete sequence of human NKLR complementary DNA; expression systems, including a CHO (chinese hamster ovarian cell line) stable expression system; and an assay using the CHO expression system.

NKlR, also known as substance P receptor, can be used in an assay to identify and evaluate entities that bind substance P receptor. The assay can also be used in conjunction with diagnosis and therapy to determine the body fluid concentration of substance P in arthritis patients.

### DETAILED DESCRIPTION OF THE INVENTION

One embodiment of the invention concerns human neurokinin-l receptor, said receptor being free of other human receptor proteins.

In one class this embodiment concerns human neurokinin-l receptor, said receptor being free of other human proteins.

Within this class, this embodiment concerns human neurokinin-l receptor from human cells such as glioblastoma, said receptor being free of other proteins.

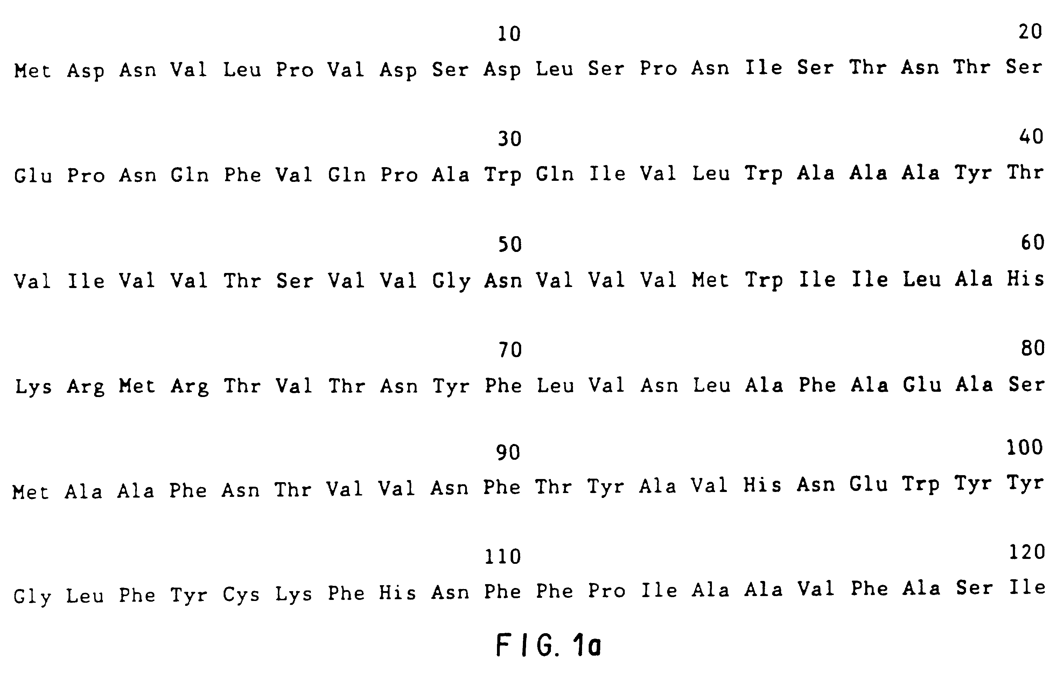

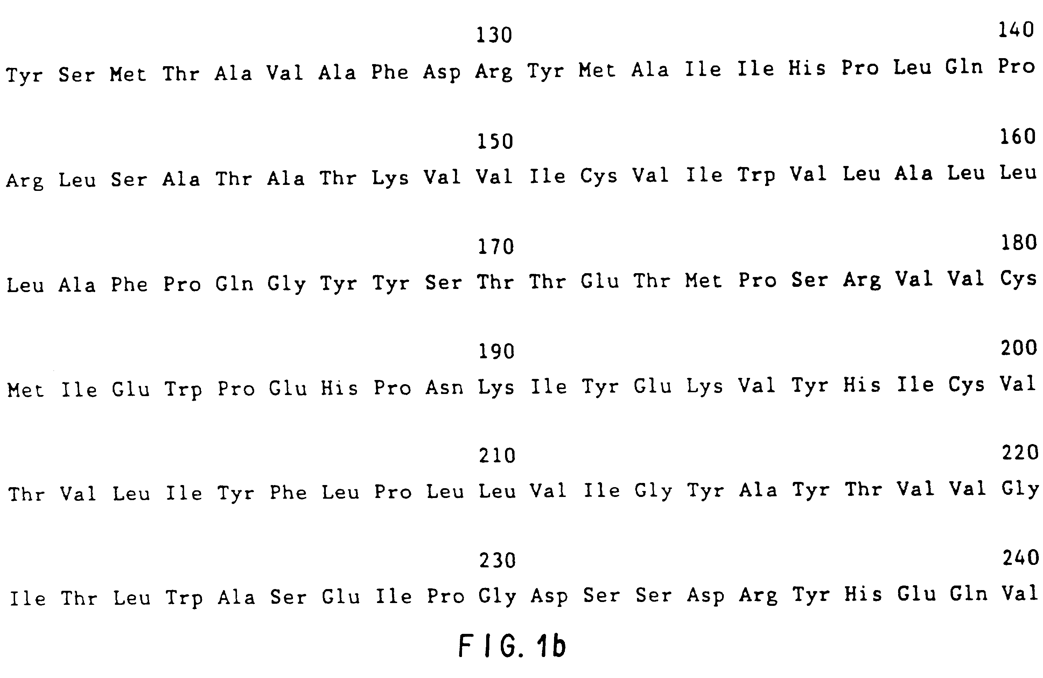

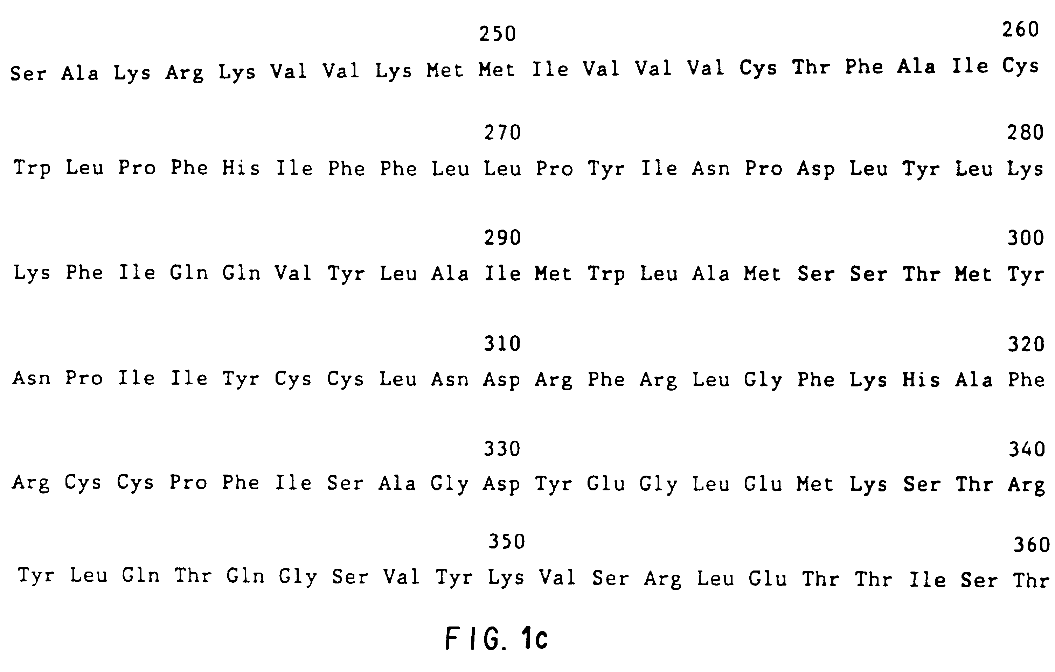

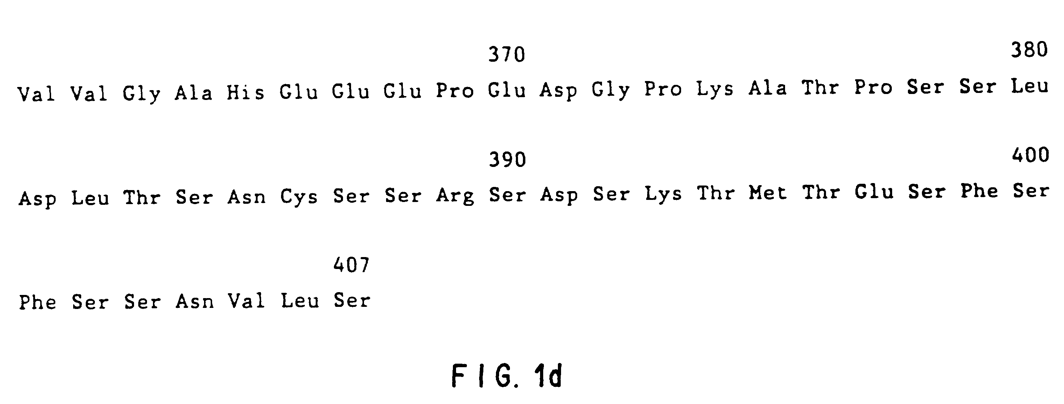

In a second class, this embodiment concerns a protein comprising the 407 amino acid sequence depicted in Figure 1, said protein being free of other human receptor proteins.

Within the second class this embodiment concerns a protein consisting of the 407 amino acid sequence as shown in Figure 1.

A second embodiment concerns a DNA sequence encoding human neurokinin receptor complementary DNA, said DNA, said sequence being free of other human DNA sequences.

As will be appreciated by those of skill in the art, there is a substantial amount of redundancy in the set of codons which translate specific amino acids. Accordingly, the invention also includes alternative base sequences wherein a codon (or codons) are replaced with another codon, such that the amino acid sequence translated by the DNA sequence remains unchanged. For purposes of this specification, a sequence bearing one or more such replaced codons will be defined as a degenerate variation. Also included are mutations (exchange of individual amino acids) which one of skill in the art would expect to have no effect on functionality, such as valine for leucine, arginine for lysine and asparigine for glutamine.

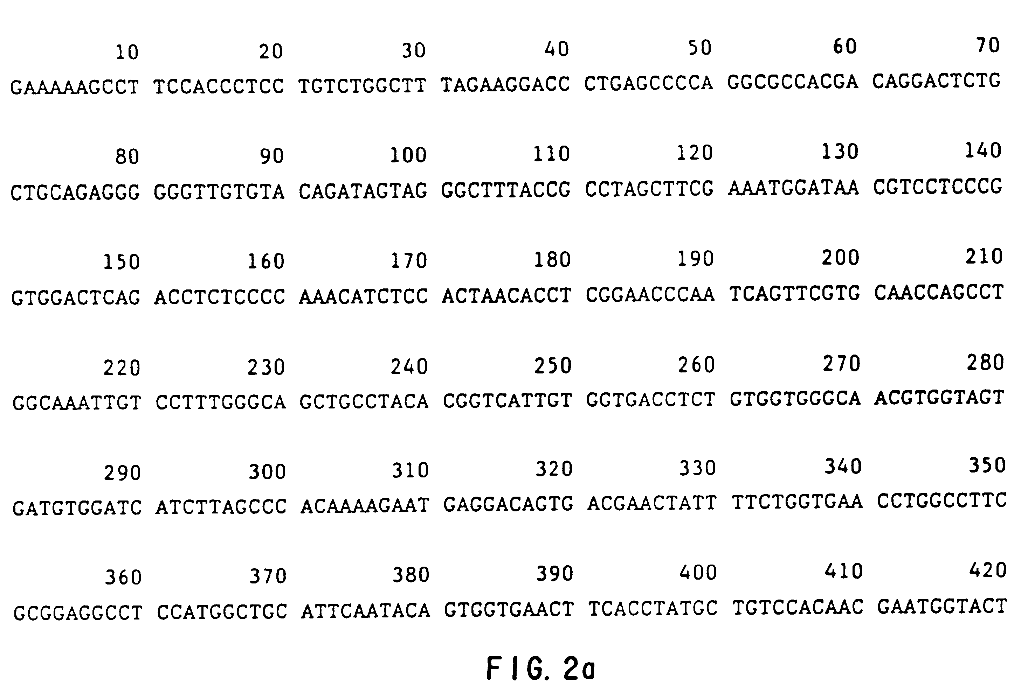

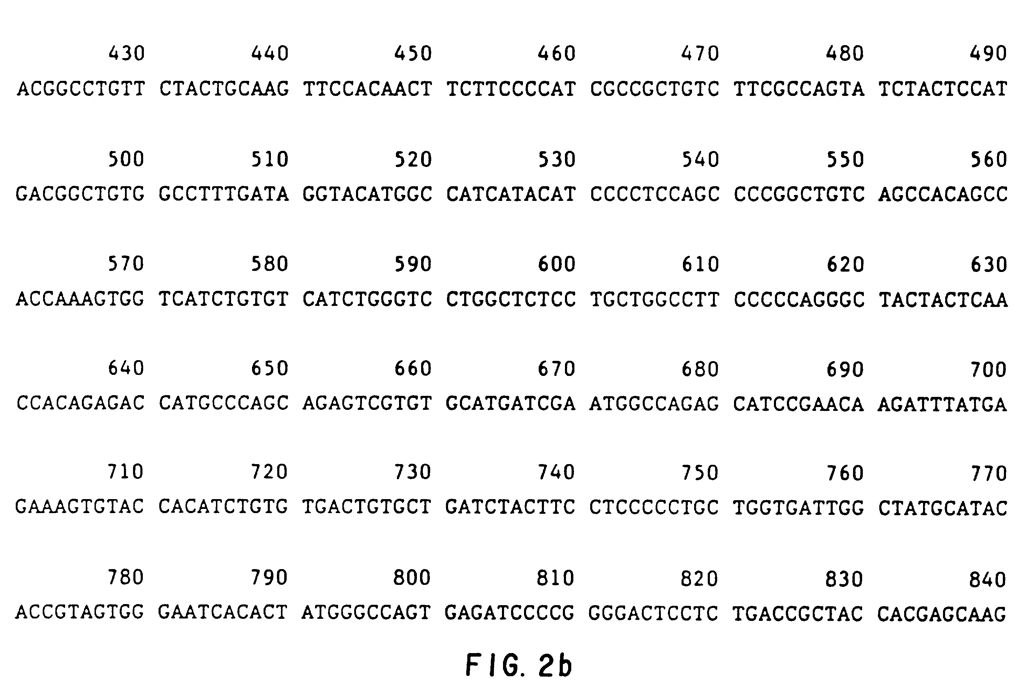

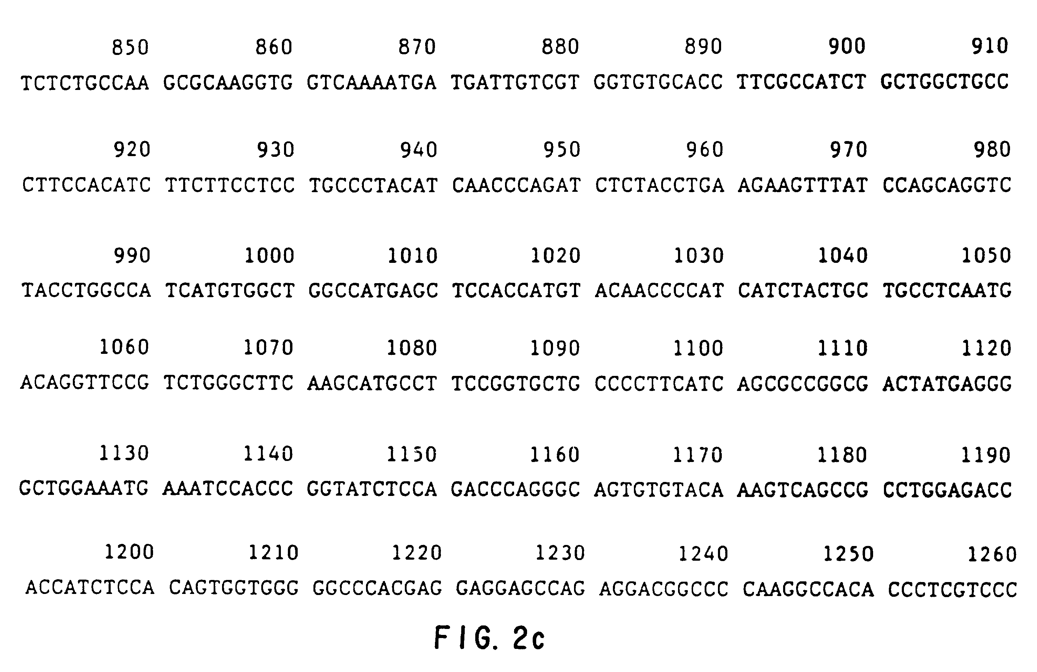

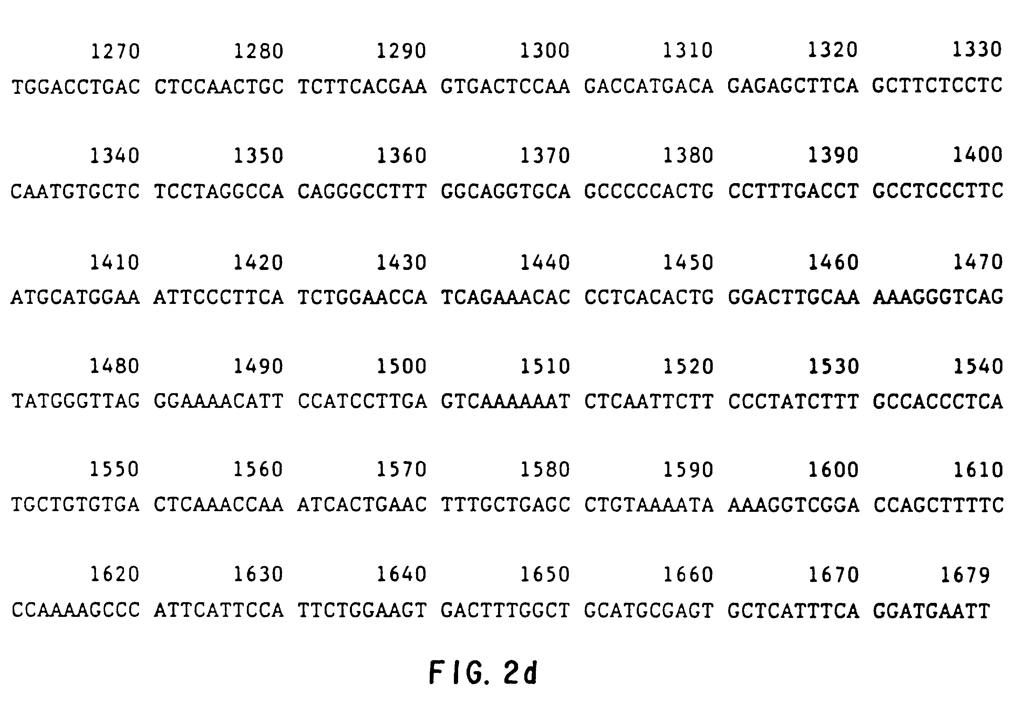

One class of the second embodiment the invention concerns the nucleotide sequence of complementary DNA, beginning with nucleotide 123 and ending with necleotide 1346 as shown in Figure 2.

Within this class of the second embodiment is the DNA sequence that further comprises: or a degenerate variation thereof.

The second embodiment the invention concerns the partial nucleotide sequence of complementary DNA, as shown in Figure 2 or a degenerate variation thereof.

A third embodiment of this invention concerns systems for expressing human neurokinin receptor.

One class this third embodiment of the invention comprises:

A plasmid which comprises:
(a) a mammalian expression vector, such as pRcCMV, and
(b) a base sequence encoding human neurokinin-l receptor protein consisting of the nucleotide sequence shown in SEQ ID NO:30, or a degenerate variation thereof.

Within this class of the third embodiment the neurokinin-1 receptor comprises the nucleotide sequence of complementary DNA, beginning with nucleotide 123 and ending with necleotide 1346 as shown in Figure 2.

A second class of this third embodiment of the invention concerns a system for the expression of human neurokinin-l receptor in a chinese hamster ovarian cell line (CHO), the system comprising a vector comprising human neurokinin receptor (NK1R) cDNA.

Within this class of the third embodiment is is the sub-class wherein the expression system includes

A plasmid which comprises:
(a) a mammalian expression vector, such as pRcCMV, and
(b) a base sequence encoding human neurokinin-1 receptor protein consisting of the nucleotide sequence shown in SEQ ID

NO:30, or a degenerate variation thereof.

Within this sub-class the neurokinin-1 receptor comprises the nucleotide sequence of complementary DNA, beginning with nucleotide 123 and ending with necleotide 1346 as shown in Figure 2. is subcloned into the vector pRcCMV.

A forth embodiment of the invention concerns a method of using any of the above expression systems for determining the binding affinity of a test sample for human neurokinin-1 receptor.

In one class this embodiment concerns a method of using a Chinese hamster ovarian cell line, said line transplanted with a plasmid, which plasmid comprises:
(a) vector pRcCMV, and
(b) the base sequence encoding human neurokinin-1 receptor protein consisting of the nucleotide sequence shown in SEQ ID NO:30, or a degenerate variation thereof, the method which comprises:
   (1) expressing human neurokinin-1 receptor in said CHO cells;
   (2) addition of a test sample to a solution containing ¹²⁵I-substance P and said cells;
   (3) incubating the products of Step (1), wherein said incubation effective for expressing said the human neurokinin-1 receptor and effective for competitive binding of said ¹²⁵I-substance P and said test sample to said human neurokinin-1 receptor;
   (4) separating said ¹²⁵I-substance P which is bound to said human neurokinin-1 receptor from said ¹²⁵I-substance P which is not bound;
   (5) measuring the radioactivity of said ¹²⁵I-substance P which is bound to said human neurokinin-1 receptor.

In a second class this embodiment concerns a method of using a Chinese hamster ovarian cell line (CHO), said line transplanted with a plasmid which plasmid comprises
(a) vector pRcCMV, and
(b) the base sequence encoding human neurokinin-1 receptor protein consisting of the nucleotide sequence shown in SEQ ID NO:30, or a degenerate variation thereof, the method comprising:
   (1) expressing human neurokinin-l receptor in said CHO cells;
   (2) equilibrating the product of Step (1) with ³H-myoinositol;
   (3) washing the product of Step (2);
   (4) incubating the product of Step (3) with a test sample in the presence of 10 mM LiCl, which results in the production of inositol monophosphate;
   (5) measuring the inositol monophosphate.

In overview, the present invention describes methods to isolate the human neurokinin-l receptor (human NKlR) complementary DNA (cDNA) without prior knowledge of its protein sequence or gene sequence. Human NKlR is a membrane receptor for the neurotransmitter substance P. Polymerase chain reaction (PCR) technique was utilized for the isolation of human NKlR cDNA. In the approach, the regions of rat NKlR Applicants thought to be similar to human NKlR were identified, oligonucleotide primers corresponding to those region were designed, PCR amplification was carried out to obtain part of the NKlR cDNA from human cells, and its DNA sequence was determined. The remaining part of the human NKlR cDNA was obtained from a human cDNA library utilizing the above sequence information of human NKlR cDNA.

The complete sequence of the human NKlR cDNA was determined, and its encoded protein sequence was deduced. Among other things, such sequence information is useful in the process of developing novel substance P antagonists.

Three heterologous expression systems were used to express the cloned human NKlR cDNA. The Xenopus oocyte expression enables one to determine the biological function of human NKlR. The COS (a monkey kidney cell line) expression can be used to measure the ligand binding properties of human NKlR. The CHO (a Chinese hamster ovarian cell line) stable expression is suitable for natural product screen to identify potential therapeutic agent or other substances that bind to substance P receptor. This cell line can also be used as an assay kit for determining the body fluid concentration of substance P in arthritis patients.

Assay protocols use the heterologously expressed human NKlR for determination of the binding affinity and antagonistic activity of substance P antagonists.

### 1) Isolation of human NKlR cDNA

To isolate the human NKlR cDNA in the absence of its sequence information, we developed methods to obtain three separate but overlapping cDNA clones in three steps. (i) We have adopted the homologous cloning strategy (Ohara et al., 1989, Proc. Nat. Acad. Sci., 86:5673-5677) to isolate cDNA clones encoding the central core region of human NKlR, with the assumption that the human NKlR sequence is similar to the published sequence (Yokota et al., 1989, J. Biol. Chem., 264:17649-17652) of rat NKlR in certain areas where appropriate PCR primers can be designed. Degenerate primers corresponding to the rat sequence were used in PCR amplification (Mullis and Faloona, 1987, Meth. Enzymol., 155:335) to obtain the cDNA encoding the central trnsmembrane core region of human NKlR from human mRNA. (ii) After determining the sequence of the core region in human NKlR, new primers corresponding to the human sequence were designed and a second homologous PCR amplification was performed using the human primer in the core region with degenerate primers corresponding to the N-terminal sequence of rat NKlR. The cDNA encoding the N-terminal region of human NKlR was thus obtained from human mRNA and its sequence was determined. (iii) An anchored PCR strategy was developed to isolate the cDNA encoding the C-terminal region of human NKlR, in which primers corresponding to the core region of human NKlR were used in combination with a primer corresponding to the sequence of a cloning vector to obtain the cDNA from a human cDNA library.

To confirm the authenticity of the cDNA encoding human NKlR, an independent PCR amplification was performed to obtain the full length cDNA in a single step using primers from the 5' and 3' untranslated regions.

### 2) Expression of the cloned human NKlR

Three expression systems were developed for the cloned human NKlR. An transient expression in Xenopus oocytes resulted from microinjection of in vitro transcribed mRNA from the cloned cDNA (Xenopus Laevis from XENOPUS ONE, Ann Arbor, MI). This system allows the measurement of biological effect of NKlR activation upon ligand binding. Another transient expression in COS (a monkey kidney cell line, ATCC CRL 1651, ATCC Rockville MD) resulted from the transfection of the cloned cDNA under the control of viral promoter into mammalian cells (e.g., COS). The transfected cells are suitable for determination binding affinity of human NKlR for various ligands. Stable expression of human NKlR in mammalian cells (e.g., CHO, a Chinese hamster ovarian cell line, ATCC CRL 9096, ATCC Rockville MD) was achieved after integration of the transfected cDNA into the chromosomes of the host cells. These stable cell lines will constituently express the cloned human NKlR and can be propagated infinitely. Therefore, stable expression system is very useful in large scale drug screen, and can be used to determine substance p concentration in the biopsy sample of patients.

To establish a stable cell line expressing the cloned human NKlR, the cDNA was subcloned into the vector pRcCMV (INVITROGEN).

The electrophysiological assay of human NKlR expressed in Xenopus oocytes was based on the fact that NKlR activates the phospholipase C upon substance P binding, and phospholipase C in turn increases the intracellular calcium concentration through inositol trisphosphate (IP₃) and IP₃-gated calcium channel on intracellular membranes. The calcium increase activates calcium-gated chloride channels on plasma membranes which gives rise to a chloride current measurable by two electrode voltage clamp.

The binding assay of human NKlR expressed in COS or CHO is based on the use of ¹²⁵I-substance P (¹²⁵I-SP, from DU PONT, Boston, MA) as a radioactively labeled ligand which compete with unlabeled substance p or any other ligand for binding to the human NKlR. Monolayer cell culture of COS or CHO was dissociated by the non-enzymatic solution (SPECIALTY MEDIA, Lavallette, NJ) and resuspended in appropriate volume of the binding buffer (50 mM Tris pH 7.5, 5 mM MnCl₂, 150 mM NaCl, 0.04 mg/ml bacitracin, 0.004 mg/ml leupeptin, 0.2 mg/ml BSA, 0.01 mM phosphoramidon) such that 200 ul of the cell suspension would give rise to about 10,000 cpm of specific ¹²⁵I-SP binding (approximately 50,000 to 200,000 cells).

The activation of phospholipase C by NKlR can also be measured in CHO cells by determining the accumulation of inositol monophosphate which is a degradation product of IP₃.

In addition to large scale drug screening using the stable CHO cell line expressing the cloned human NKlR, other alternative applications are obvious. For example, the stable cell line can be used in the binding assay to determine the substance p concentration from biopsy samples. The human NKlR protein can also be injected into patients to reduce substance P concentration in some neurogenic inflammatory diseases.

### EXAMPLE 1

### Step A:

In the first step of obtaining the cDNA encoding the central core region of human NKlR, human mRNA was prepared from three human glioblastoma cell lines T98G, CCF-STTG1 and U87MG (obtained from the American Type Culture Collection, Rockville, MD) by the FASTTRACK method (INVITROGEN, San Diego, CA). Synthesis of first strand cDNA from 4 ug of human mRNA was initiated by oligo (dT) primers in a total volume of 20 ul according to protocols of the BRL cDNA synthesis system (BRL, LIFE TECHNOLOGIES, Inc., Gaithersburg, MD). Ten ul of the first strand cDNA was used as template with three rat primers (50 pmol rspr2s4, 50 pmol rspr2s4h, and 100 pmol rspr7a2; see Table I for their sequences) in a primary PCR amplification in a total volume of 100 ul according to the GENEAMP protocol (PERKIN ELMER CETUS, Norwalk, CT). Thirty cycles of PCR were performed using the following parameters: 1 min of denaturation at 94°C, 2 min of annealing at 40°C and 4 min of extension at 72°C with 2 sec of auto extension. Ten ul of the primary PCR product was used as template with the same primers in a secondary PCR amplification under the same cycling conditions to further amplify the DNA. Ten ul of the secondary PCR product was used as template with three rat primers (50 pmol rspr2s4, 50 pmol rspr2s4h, 50 pmol rspr7al and 50 pmol rspr7alh) in 30 cycles of tertiary PCR amplification with the following parameters: 1 min of denaturation at 94°C, 2 min of annealing at 45°C, and 4 min of extension at 72°C with 2 sec of auto extension. The tertiary PCR product was analyzed by agarose gel electrophoresis and was found to contain a 600 bp DNA fragment. This DNA fragment was excised from the gel, purified by GENECLEAN (Bio 101, La Jolla, CA), phosphorylated, and subcloned into Sma I site of the plasmid vector BLUESCRIPT SK+ (STRATAGENE, La Jolla, CA). The DNA sequence was determined by the Sequenase dideoxy chain termination method (USBC, Cleveland, OH). Sequence alignment analysis showed that this cDNA fragment is similar (90% identity at nucleotide level) to the central core region of rat NKlR from amino acid 91 to 280.

### STEP B:

After determination of the core region sequence of human NKlR, five antisense primers were synthesized based on the human sequence (hspr3a5, hspr5a1, hspr5a2, hspr6a1 and hspr6a2; see Table II for their sequences). These primers would be used to obtain the N-terminal cDNA sequence of human NKlR. One ug of human glioblastoma mRNA and 6 uM of each of the above primers was used in first strand cDNA synthesis in a total volume of 20 ul according the BRL cDNA synthesis protocols. The cDNA was extracted by phenol-chloroform, precipitated by ethanol and dissolved in 30 ul of water. Ten ul of the cDNA was used as template with two rat primers (50 pmol rsprn and 50 pmol rsprnh) and one human primer (150 pmol hspr3a5) in the primary PCR amplification in a total volume of 100 ul. Thirty cycles were performed with the following parameters: 1 min denaturation at 94°C, 1 min of annealing at 55°C, and 3 min of extension at 72°C. Five ul of the primary PCR product was then used as template with two rat primers (50 pmol rsprn and 50 pmol rsprnh) and one human primer (100 pmol hspr3a4) in 30 cycles of secondary PCR amplification with the same parameters. Two ul of the secondary PCR product was used as template with two rat primers (50 pmol rsprn and 50 pmol rsprnh) and one human primer in 30 cycles of tertiary PCR amplification with the same parameters. The tertiary PCR product was analyzed by agarose gel electrophoresis and was found to contain a 500 bp fragment. This DNA fragment can hybridize with a human oligonucleotide (hspr3a2), indicating it is not a non-specific by-product. This DNA fragment was excised from the gel, purified by GENECLEAN (Bio 101), phosphorylated, and subcloned into Sma I site of the vector Bluescript SK+. DNA sequence analysis revealed that this fragment encodes the human NKlR N-terminal region and it also contains 5' untranslated sequence.

### STEP C:

In the third step, an anchored PCR protocol was developed in which the cDNA encoding the C-terminal region of human NKlR was obtained from a cDNA library using sense human primers and a primer corresponding to the vector sequence. Three ug of human glioblastoma mRNA was primed by 2.5 ug of oligo (dT) in the first strand cDNA synthesis in a total volume of 50 ul, followed by second strand cDNA synthesis according the BRL cDNA synthesis protocols. The cDNA product was then heated at 70°C for 10 min. The yield of double stranded cDNA was determined by incorporating 1.25 uM of ³²P-α-dCTP as tracer in the reaction. Four ul of T4 DNA polymerase was added to the reaction mixture and incubated at 37°C for 10 min. The reaction was stopped by adding 16 ul of 250 mM EDTA, extracting with phenol/CHCl₃, and precipitating with ethanol. The cDNA was dissolved in 50 ul of HE buffer (10 mM HEPES-1mM EDTA). Small size cDNA was removed by the Select-D(RF) SPIN COLUMN (5'TO3', Boulder, CO), and the large size cDNA was precipitated by ethanol and dissolved in 36 ul of water. Four ul of 0.2 M Tris-10 mM spermidine-1 mM EDTA (pH7.5) was added to the tube and heated at 70°C for 1 min. The cDNA was phosphorylated by adding 5 ul of blunt-end kinase buffer (0.5 M Tris pH 9.5, 0.1 M MgCl₂, 50 mM DTT, 50% glycerol), 2.5 ul of 10 mM ATP, 2.5 ul of polynucleotide kinase, and incubating at 37°C for 30 min. The cDNA was extracted by phenol/CHCl₃, precipitated by ethanol and ligated to EcoRI linker according to the PROMEGA ECORI linker ligation protocol (PROMEGA, Madison, WI). Linker-ligated cDNA was then ligated to calf intestinal phosphatase-treated EcoRI site of the vector BLUESCRIPT SK+. One ul of the ligated plasmid DNA was used as template in 30 cycles of primary PCR with two human primers (50 pmol hspr6s1 and 50 pmol hspr6s2) and 100 pmol of vector-specific primer t3 (obtained from STRATAGENE) with the following parameters: 1 min of denaturation at 94°C, 2 min of annealing at 55°C, and 4 min of extension at 72°C with 2 sec auto extension. One ul of the primary PCR product was used in 30 cycles of secondary PCR amplification with one human primer (100 pmol hspr6s3) and the same vector-specific primer t3 under the same conditions. One ul of the secondary PCR product was used in 30 cycles of tertiary PCR amplification with one human primer (100 pmol hspr6s4) and 100 pmol of vector-specific primer SK (STRATAGENE) under the same conditions. A 780 bp DNA fragment was detected which also hybridized to a human oligo probe hspr6s5. This DNA fragment was excised from the agarose gel, purified by GENECLEAN (BIO 101), phosphorylated, and subcloned into Sma I site of the vector BLUESCRIPT SK+. DNA sequence analysis revealed that it encodes the C-terminal region of human NKlR and contains 3' untranslated sequence.

### STEP D:

Since three separate but overlapping cDNA clones encoding human NKlR were isolated above and the possibility of alternative pre-mRNA splicing exists, it is necessary to confirm the authenticity of the full length cDNA sequence by isolating a full length cDNA directly. Based on the above sequence in the untranslated region, primers were synthesized which should give rise to a full length cDNA. Using the PERKIN ELMER CETUS RNA PCR amplification kit (Perkin Elmer Cetus), cDNA was synthesized from 1.5 ug of human glioblastoma mRNA in a total volume of 20 ul with 50 pmol of the human primer hspr3uta5. One half of the first strand cDNA was used as template in 30 cycles of primary PCR amplification with two human primers (50 pmol hspr3uta5, 50 pmol hspr5uts1) with the following parameters: 1 min of denaturation at 94°C, 2 min of annealing at 55°C, and 4 min of extension at 55°C with 2 sec auto extension. Ten ul of the primary PCR product was used as template in 30 cycles of secondary PCR amplification with two human primers (50 pmol hspr3uta6 and 50 pmol hspr5uts2) under the same conditions. A 1350 bp DNA fragment was excised from agarose gel, purified by GENECLEAN (BIO 101), digested with restriction endonucleases with EcoRI and Not I, and subcloned into the vector BLUESCRIPT SK+. DNA sequence analysis confirmed the general structure of the cloned human NKlR cDNA. The sequence of human NKlR CDNA is shown in Figure 1.

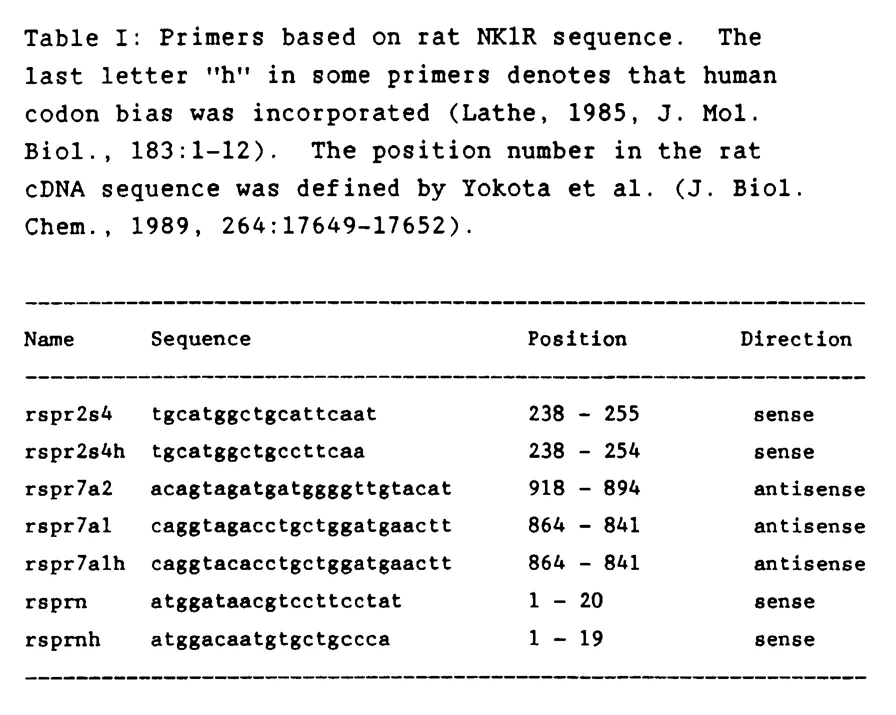

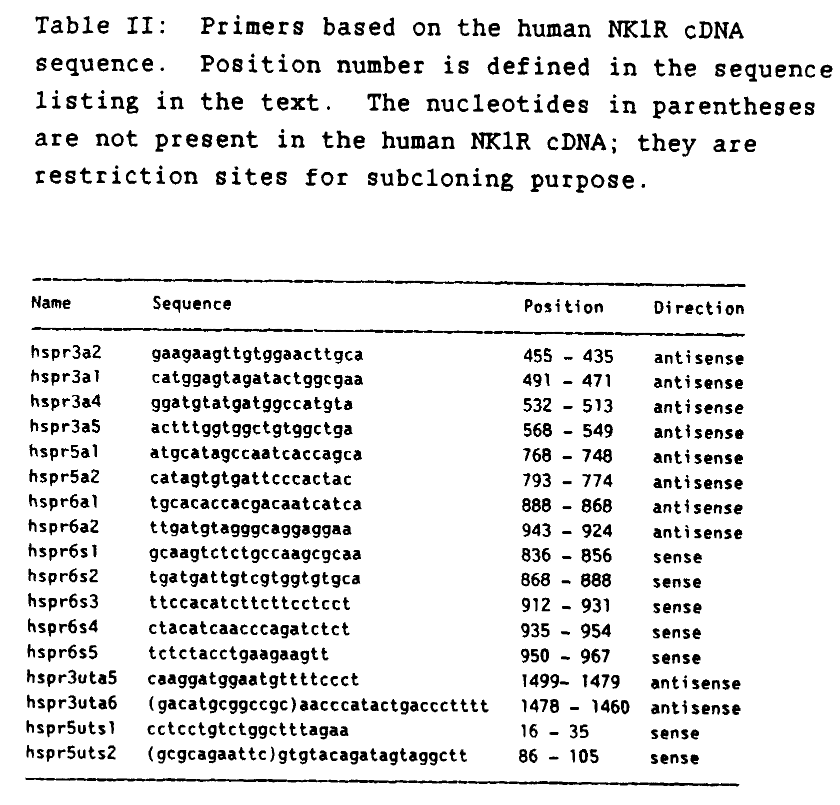

### Expression in Xenopus oocytes

To express the human NK1R cDNA in Xenopus oocytes, the cDNA was cloned into an in vitro transcription vector BLUESCRIPT SK+ (STRATAGENE) which contains the T7 promoter for initiation of T7 RNA polymerase catalyzed RNA synthesis. One ug of linear plasmid DNA which contained the human NK1R cDNA downstream of the T7 promoter was used in the in vitro transcription reaction containing 40 mM Tris pH 7.5, 50 mM NaCl, 8 mM MgCl₂, 2 mM spermidine, 0.4 mM CTP, 0.4 mM ATP, 0.4 mM UTP, 0.16 mM GTP, 2.5 ul CAP analog (STRATAGENE), 30 mM DTT, 1 U RNase Block II (STRATAGENE), 0.83 pmol ³²P-α-CTP and 25 U of T7 RNA polymerase. The reaction tube was incubated at 37°C for 1 hour. Usually 5 ug of RNA was synthesized as quantitated by incorporation of ³²P-α-CTP into RNA. After RNA synthesis, the plasmid DNA was removed by adding 10 U of RNase free DNase and 1 U of RNase Block II. The reaction mixture was extracted by phenol/CHCl₃, and the unincorporated nucleotides were removed by the Select-D(RF) spin column (5'T03'). The RNA transcript was precipitated by ethanol twice and dissolved in RNase free water. Oocytes were removed from Xenopus frogs, treated with 2 mg/ml collagenase (specific activity < 0.3 U/mg, BOEHRINGER MANNHEIM, Indianapolis, IN) in OR-2 buffer (82.5 mM NaCl, 2 mM KCl, 1 mM MgCl₂, 5 mM HEPES, pH 7.4) for 4 hours at 19°C. The dissociated oocytes were incubated in OR-2 buffer supplemented by 1.8 mM CaCl₂, 0.5 mg/ml gentamycin and 0.5 mM theophylline at 19°C overnight before injection. A 50 nl aliquot contain 2 ng of RNA transcript was injected into each oocyte. The injected oocytes were incubated at 19°C for 2 days before electrophysiological recording (see Example 3 for assay method).

### Expression in COS

To express the human NKlR transiently in COS, the cDNA was cloned into the expression vector pCDM9 which was derived from pCDM8 (INVITROGEN) by inserting the ampicillin resistance gene (nucleotide 1973 to 2964 from BLUESCRIPT SK+) into the Sac II site. Transfection of 20 ug of the plasmid DNA into 10 millions COS cells was achieved by electroporation in 800 ul of transfection buffer (135 mM NaCl, 1.2 mM CaCl₂, 1.2 mM MgCl₂, 2.4 mM K₂HPO₄, 0.6 mM KH₂PO₄, 10 mM glucose, 10 mM HEPES pH 7.4) at 260 V and 950 uF using the IBI GENEZAPPER (IBI, New Haven, CT). The cells were incubated in 10% fetal calf serum, 2 mM glutamine, 100U/ml penicillin-streptomycin, and 90% DMEM media (GIBCO, Grand Island, NY) in 5% CO₂ at 37°C for three days before the binding assay.

### Stable Expression in CHO

To establish a stable cell line expressing the cloned human NKlR, the cDNA was subcloned into the vector pRcCMV (INVITROGEN). Transfection of 20 ug of the plasmid DNA into CHO cells was achieved by electroporation in 800 ul of transfection buffer suplemented with 0.625 mg/ml Herring sperm DNA at 300 V and 950 uF using the IBI GENEZAPPER (IBI). The transfected cells were incubated in CHO media [10 % fetal calf serum, 100 U/ml pennicilin-streptomycin, 2 mM glutamine, 1/500 hypoxanthine-thymidine (ATCC), 90% IMDM media (JRH BIOSCIENCES, Lenexa, KS), 0.7 mg/ml G418 (GIBCO)] in 5% CO₂ at 37°C until colonies were visible. Each colony was separated and propagated. The cell clone with the highest number of human NKlR was selected for subsequent application in the assay of Example 3.

### Example 3

### Assay Protocol Using Oocytes

The oocyte was voltage-clamped at - 80 mV by the model 8500 intracellular preamp-clamp (DAGAN, Minneapolis, MN). The recoding chamber was continuously perfused with recording buffer (96 mM NaCl, 2 mM KCl, 1.8 mM CaCl₂, 5 mM HEPES, pH 7.4). Chloride current was elicited by applying substance P (from 0.1 nM to 1000 nM) to the recording chamber. At least three oocytes were measured for each concentration. The antagonistic activity of any potential substance P antagonist can be assessed by determining the inhibition of substance P response. Likewise, NK1 agonists can be identified by their ability to stimulate a response in oocytes injected with NKlR mRNA but not in uninjected oocytes.

### Assay Protocol using COS or CHO

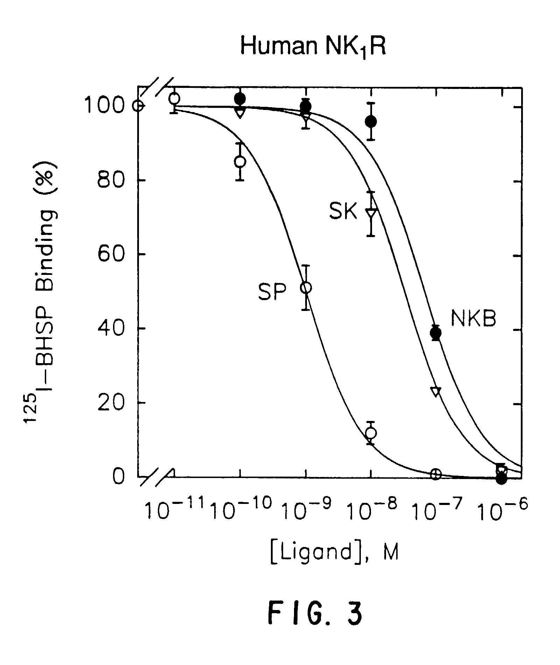

The binding assay of human NKlR expressed in COS or CHO is based on the use of ¹²⁵I-substance P (¹²⁵I-SP, from DU PONT, Boston, MA) as a radioactively labeled ligand which compete with unlabeled substance p or any other ligand for binding to the human NKlR. Monolayer cell culture of COS or CHO was dissociated by the non-enzymatic solution (SPECIALTY MEDIA, Lavallette, NJ) and resuspended in appropriate volume of the binding buffer (50 mM Tris pH 7.5, 5 mM MnCl₂, 150 mM NaCl, 0.04 mg/ml bacitracin, 0.004 mg/ml leupeptin, 0.2 mg/ml BSA, 0.01 mM phosphoramidon) such that 200 ul of the cell suspension would give rise to about 10,000 cpm of specific ¹²⁵I-SP binding (approximately 50,000 to 200,000 cells). In the binding assay, 200 ul of cells were added to a tube containing 20 ul of 1.5 to 2.5 nM of ¹²⁵I-SP and 20 11 of unlabeled substance p or any other test compound. The tubes were incubated at 4°C or at room temperature for 1 hour with gentle shaking. The bound radioactivity was separated from unbound radioactivity by GF/C filter (BRANDEL, Gaithersburg, MD) which was pre-wetted with 0.1 % polyethylenimine. The filter was washed with 3 ml of wash buffer (50 mM Tris pH 7.5, 5 mM MnCl₂, 150 mM NaCl) three times and its radioactivity was determined by gamma counter. Illustrative of this method of using these expression systems are the results shoen in Figure 3. These results show the competitive binding of substance P (SP), substance K (SK) and human neurokinin-1 receptor (NKlR) in the COS assay.

### ALTERNATIVE PROTOCOL

The activation of phospholipase C by NKLR can also be measured in CHO cells by determining the accumulation of inositol monophosphate which is a degradation product of IP₃. CHO cells were seeded in 12-well plate at 250,000 cells per well. After incubating in CHO media for 4 days, cells were loaded with 0.025 uCi/ml of ³H-myoinositol by overnight incubation. The extracellular radioactivity was removed by washing with phosphate buffered saline. LiCl was added to the well at final concentration of 0.1 mM with or without antagonist, and continued incubation at 37°C for 15 min. Substance p was added to the well at final concentration of 0.3 nM to activate the human NKlR. After 30 min of incubation at 37°C, the media was removed and 0.1 N HCl was added. Each well was sonicated at 4°C and extracted with CHCl₃/methanol (1:1). The aqueous phase was applied to a 1 ml Dowex AG 1X8 ion exchange column. The column was washed with 0.1 N formic acid followed by 0.025 M ammonium formate-0.1 N formic acid. The inositol monophosphate was eluted with 0.2 M ammonium formate-0.1 N formic acid and quantitated by beta counter.

## Claims

1. A human neurokinin-l receptor corresponding to the amino acid sequence which is: the receptor being substantially free of other human receptor proteins.

2. A DNA sequence, encoding human neurokinin-1 receptor, consisting of the nucleotide sequence shown in SEQ ID NO:30, or a degenerate variation thereof, the sequence being free of other human DNA sequences.

3. A DNA sequence encoding human neurokinin-1 receptor comprising the sequence which is: or a degenerate variation thereof.

4. The DNA sequence of Claim 3 further comprising the sequence which is: or a degenerate variation thereof.

5. The DNA sequence of Claim 4 further comprising the sequence which is: or a degenerate variation thereof.

6. A plasmid which comprises:
(a) a mammalian expression vector, and
(b) a base sequence encoding human neurokinin-1 receptor protein consisting of the nucleotide sequence shown in SEQ ID NO:30, or a degenerate variation thereof.

7. A method for determining the binding affinity of a test sample for human neurokinin-1 receptor by using a Chinese hamster ovarian cell line (CHO), the cell line transfected with a plasmid, which plasmid comprises:
(a) a mammalian expression vector, and
(b) a base sequence encoding human neurokinin-1 receptor protein, consisting of the nucleotide sequence shown in SEQ ID NO:30, or a degenerate variation thereof,
the method which comprises:
(1) expressing human neurokinin-l receptor in the CHO cells;
(2) adding a test sample to a solution containing ¹²⁵I-substance P and the CHO cells;
(3) incubating the products of Step 2, wherein the incubation is effective for competitive binding of the ¹²⁵I-substance P and the test sample to the human neurokinin-1 receptor;
(4) separating the ¹²⁵I-substance P which is bound to the human neurokinin-1 receptor from the ¹²⁵I-substance P which is not bound;
(4) measuring the amount of the ¹²⁵I-substance P which is bound to the human neurokinin-1 receptor.

8. A method for determining the binding affinity of a test sample for human neurokinin-1 receptor by using a Chinese hamster ovarian cell line (CHO), the line transfected with a plasmid, which plasmid comprises:
(a) a mammalian expression vector, and
(b) a base sequence encoding human neurokinin-1 receptor protein, consisting of the nucleotide sequence shown in SEQ ID NO:30, or a degenerate variation thereof,
the method which comprises:
(1) expressing human neurokinin-l receptor in the CHO cells;
(2) equilibrating the product of Step (1) with ³H-myoinositol;
(3) washing the product of Step (2);
(4) incubating the product of Step (3) with a test sample in the presence of aqueous LiCl, resulting in the production of ³H-inositol monophosphate;
(5) measuring the ³H-inositol monophosphate.

## Patentansprüche

1. Human-Neurokinin-1-Rezeptor, entsprechend der folgenden Aminosäuresequenz: wobei der Rezeptor im wesentlichen frei von anderen Human-Rezeptorproteinen ist.

2. DNA-Sequenz, welche für einen Human-Neurokinin-1-Rezeptor kodiert, bestehend aus der in SEQ-ID-Nr. 30 dargestellten Nukleotidsequenz oder einer Degenerationsvariante davon, wobei die Sequenz frei von anderen Human-DNA-Sequenzen ist.

3. DNA-Sequenz, welche für einen Human-Neurokinin-1-Rezeptor kodiert, umfassend die folgende Sequenz: oder eine Degenerationsvariante davon.

4. DNA-Sequenz nach Anspruch 3, umfassend ferner die folgende Sequenz: oder eine Degenerationsvariante davon.

5. DNA-Sequenz nach Anspruch 4, umfassend ferner die folgende Sequenz: oder eine Degenerationsvariante davon.

6. Plasmid, welches umfaßt:
(a) einen Säuger-Expressionsvektor, und
(b) eine Basensequenz, die für ein Human-Neurokinin-1-Rezeptorprotein kodiert, bestehend aus der in SEQ-ID-Nr. 30 dargestellten Nukleotidsequenz oder einer Degenerationsvariante davon.

7. Verfahren zur Bestimmung der Bindungsaffinität einer Testprobe für Human-Neurokinin-1-Rezeptor unter Verwendung einer Eierstock-Zellinie des Chinesischen Hamsters (CHO), wobei die Zellinie mit einem Plasmid transfiziert ist, welches Plasmid umfaßt:
(a) einen Säuger-Expressionsvektor, und
(b) eine Basensequenz, die für ein Human-Neurokinin-1-Rezeptorprotein kodiert, bestehend aus der in SEQ-ID-Nr. 30 dargestellten Nukleotidsequenz oder einer Degenerationsvariante davon,
welches Verfahren umfaßt:
(1) Expression von Human-Neurokinin-1-Rezeptor in den CHO-Zellen;
(2) Zugabe einer Testprobe zu einer Lösung, die ¹²⁵I-Substanz P und die CHO-Zellen enthält;
(3) Inkubation der Produkte von Schritt 2, wobei die Inkubation wirksam ist für die konkurrierende Bindung der ¹²⁵I-Substanz P und der Testprobe an den Human-Neurokinin-1-Rezeptor;
(4) Trennung der an den Human-Neurokinin-1-Rezeptor gebundenen ¹²⁵I-Substanz P von der nicht gebundenen ¹²⁵I-Substanz P;
(5) Messung der Menge der an den Human-Neurokinin-1-Rezeptor gebundenen ¹²⁵I-Substanz P.

8. Verfahren zur Bestimmung der Bindungsaffinität einer Testprobe für Human-Neurokinin-1-Rezeptor unter Verwendung einer Eierstock-Zellinie des Chinesischen Hamsters (CHO), wobei die Zellinie mit einem Plasmid transfiziert ist, welches Plasmid umfaßt:
(a) einen Säuger-Expressionsvektor, und
(b) eine Basensequenz, die für ein Human-Neurokinin-1-Rezeptorprotein kodiert, bestehend aus der in SEQ-ID-Nr. 30 dargestellten Nukleotidsequenz oder einer Degenerationsvariante davon,
welches Verfahren umfaßt:
(1) Expression von Human-Neurokinin-1-Rezeptor in den CHO-Zellen;
(2) Äquilibrierung des Produkts von Schritt (1) mit ³H-Myoinosit;
(3) Waschen des Produkts von Schritt (2);
(4) Inkubation des Produkts von Schritt (3) mit einer Testprobe in Anwesenheit von wäßrigem LiCl, was zur Bildung von ³H-Inositmonophosphat führt;
(5) Messung des ³H-Inositmonophosphats.

## Revendications

1. Récepteur de la neurokinine-1 humain correspondant à la séquence d'acides aminés qui est : le récepteur étant pratiquement exempt d'autres protéines de récepteur humaines.

2. Séquence d'ADN, codant pour le récepteur de la neurokinine-1 humain, constituée de la séquence nucléotidique représentée dans SEQ ID N° 30, ou une variation dégénérée de celle-ci, la séquence étant exempte d'autres séquences d'ADN humain.

3. Séquence d'ADN codant pour le récepteur de la neurokinine-1 humain comportant la séquence qui est : ou une variation dégénérée de celle-ci.

4. Séquence d'ADN selon la revendication 3, comportant de plus la séquence qui est : ou une variation dégénérée de celle-ci.

5. Séquence d'ADN selon la revendication 4, comportant de plus la séquence qui est : ou une variation dégénérée de celle-ci.

6. Plasmide qui comporte :
(a) un vecteur d'expression de mammifère, et
(b) une séquence de base codant pour la protéine du récepteur de la neurokinine-1 humain constituée de la séquence nucléotidique représentée dans la SEQ ID N° 30, ou une variation dégénérée de celle-ci.

7. Procédé de détermination de l'affinité de liaison d'un échantillon test du récepteur de la neurokinine-1 humain par l'utilisation d'une lignée cellulaire d'ovaire d'hamster chinois (CHO), lignée cellulaire transfectée à l'aide d'un plasmide, plasmide qui comporte :
(a) un vecteur d'expression de mammifère, et
(b) une séquence de base codant pour la protéine du récepteur de la neurokinine-1 humain, constituée de la séquence nucléotidique représentée dans la SEQ ID N° 30, lequel procédé consiste à :
(1) exprimer le récepteur de la neurokinine-1 humain dans les cellules CHO ;
(2) ajouter un échantillon test à une solution contenant une substance P-I¹²⁵ et les cellules CHO ;
(3) mettre à incuber les produits de l'Etape (2), où l'incubation est efficace pour une liaison compétitive de la substance P-I¹²⁵ et de l'échantillon test au récepteur de la neurokinine-1 humain ;
(4) séparer la substance P-I¹²⁵ qui est liée au récepteur de la neurokinine-1 humain de la substance P-I¹²⁵ qui n'est pas liée ;
(5) mesurer la quantité de la substance P-I¹²⁵ qui est liée au récepteur de la neurokinine-1 humain.

8. Procédé de détermination de l'affinité de liaison d'un échantillon test pour le récepteur de la neurokinine-1 humain en utilisant une lignée cellulaire d'ovaire d'hamster chinois (CHO), ladite lignée étant transfectée par un plasmide, lequel plasmide comporte :
(a) un vecteur d'expression de mammifère, et
(b) une séquence de base codant pour la protéine du récepteur de la neurokinine-1 humain, constituée de la séquence nucléotidique représentée dans la SEQ ID N° 30, ou une variation dégénérée de celle-ci, lequel procédé consiste à :
(1) exprimer le récepteur de la neurokinine-1 humain -dans les cellules CHO ;
(2) équilibrer le produit de l'Etape (1) à l'aide de myoinositol-H³ ;
(3) laver le produit de l'Etape (2) ;
(4) mettre à incuber le produit de l'Etape (3) avec un échantillon test en présence de LiCl aqueux, ayant pour résultat la production d'inositol-H³ monophosphate ;
(5) mesurer l'inositol-H³ monophosphate.


### SEQUENCE LISTING

### (2) INFORMATION FOR SEQ ID NO:1:

(i) SEQUENCE CHARACTERISTICS:
   (A) LENGTH: 122 base pairs
   (B) TYPE: nucleic acid
   (C) STRANDEDNESS: single
   (D) TOPOLOGY: linear
(ii) MOLECULE TYPE: cDNA to mRNA
(xi) SEQUENCE DESCRIPTION: SEQ ID NO:1:
   GAAAAAGCCT TCCACCCTCC TGTCTGGCTT TAGAAGGACC CTGAGCCCCA    50
   GGCGCCACGA CAGGACTCTG CTGCAGAGGG GGGTTGTGTA CAGATAGTAG   100
   GGCTTTACCG CCTAGCTTCG AA                                 122

### (2) INFORMATION FOR SEQ ID NO:2:

(i) SEQUENCE CHARACTERISTICS:
   (A) LENGTH: 18 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:2:
   TGCATGGCTG CATTCAAT    18

### (2) INFORMATION FOR SEQ ID NO:3:

(i) SEQUENCE CHARACTERISTICS:
   (A) LENGTH: 17 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:3:
   TGCATGGCTG CCTTCAA    17

### (2) INFORMATION FOR SEQ ID NO:4:

(i) SEQUENCE CHARACTERISTICS:
   (A) LENGTH: 24 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:4:
   ACAGTAGATG ATGGGGTTGT ACAT 24

### (2) INFORMATION FOR SEQ ID NO:5:

(i) SEQUENCE CHARACTERISTICS:
   (A) LENGTH: 24 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:5:
   CAGGTAGACC TGCTGGATGA ACTT 24

### (2) INFORMATION FOR SEQ ID NO:6:

(i) SEQUENCE CHARACTERISTICS:
   (A) LENGTH: 24 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:6:
   CAGGTACACC TGCTGGATGA ACTT 24

### (2) INFORMATION FOR SEQ ID NO:7:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:7:
   ATGGATAACG TCCTTCCTAT 20

### (2) INFORMATION FOR SEQ ID NO:8:

(i) SEQUENCE CHARACTERISTICS:
   (A) LENGTH: 19 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:8:
   ATGGACAATG TGCTGCCCA 19

### (2) INFORMATION FOR SEQ ID NO:9:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:9:
   GAAGAAGTTG TGGAACTTGC A 21

### (2) INFORMATION FOR SEQ ID NO:10:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:10:
   CATGGAGTAG ATACTGGCGA A 21

### (2) INFORMATION FOR SEQ ID NO:11:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:11:
   GGATGTATGA TGGCCATGTA 20

### (2) INFORMATION FOR SEQ ID NO:12:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:12:
   ACTTTGGTGG CTGTGGCTGA 20

### (2) INFORMATION FOR SEQ ID NO:13:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:13:
   ATGCATAGCC AATCACCAGC A 21

### (2) INFORMATION FOR SEQ ID NO:14:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:14:
   CATAGTGTGA TTCCCACTAC 20

### (2) INFORMATION FOR SEQ ID NO:15:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:15:
   TGCACACCAC GACAATCATC A 21

### (2) INFORMATION FOR SEQ ID NO:16:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:16:
   TTGATGTAGG GCAGGAGGAA 20

### (2) INFORMATION FOR SEQ ID NO:17:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:17:
   GCAAGTCTCT GCCAAGCGCA A 21

### (2) INFORMATION FOR SEQ ID NO:18:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:18:
   TGATGATTGT CGTGGTGTGC A 21

### (2) INFORMATION FOR SEQ ID NO:19:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:19:
   TTCCACATCT TCTTCCTCCT 20

### (2) INFORMATION FOR SEQ ID NO:20:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:20:
   CTACATCAAC CCAGATCTCT 20

### (2) INFORMATION FOR SEQ ID NO:21:

(i) SEQUENCE CHARACTERISTICS:
   (A) LENGTH: 18 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:21:
   TCTCTACCTG AAGAAGTT 18

### (2) INFORMATION FOR SEQ ID NO:22:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:22:
   CAAGGATGGA ATGTTTTCCC T 21

### (2) INFORMATION FOR SEQ ID NO:23:

(i) SEQUENCE CHARACTERISTICS:
   (A) LENGTH: 32 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:23:
   GACATGCGGC CGCAACCCAT ACTGACCCTT TT 32

### (2) INFORMATION FOR SEQ ID NO:24:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:24:
   CCTCCTGTCT GGCTTTAGAA 20

### (2) INFORMATION FOR SEQ ID NO:25:

(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:25:
   GCGCAGAATT CGTGTACAGA TAGTAGGCTT 30

### (2) INFORMATION FOR SEQ ID NO:26:

(i) SEQUENCE CHARACTERISTICS:
   (A) LENGTH: 1224 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:26:

### (2) INFORMATION FOR SEQ ID NO:27:

(i) SEQUENCE CHARACTERISTICS:
   (A) LENGTH: 333 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:27:

### (2) INFORMATION FOR SEQ ID NO:28:

(i) SEQUENCE CHARACTERISTICS:
   (A) LENGTH: 1346 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:28:
   GAAAAAGCCT TCCACCCTCC TGTCTGGCTT TAGAAGGACC CTGAGCCCCA GGCGCCACGA     60
   CAGGACTCTG CTGCAGAGGG GGGTTGTGTA CAGATAGTAG GGCTTTACCG CCTAGCTTCG    120
   AAATGGATAA CGTCCTCCCG GTGGACTCAG ACCTCTCCCC AAACATCTCC ACTAACACCT    180
   CGGAACCCAA TCAGTTCGTG CAACCAGCCT GGCAAATTGT CCTTTGGGCA GCTGCCTACA    240
   CGGTCATTGT GGTGACCTCT GTGGTGGGCA ACGTGGTAGT GATGTGGATC ATCTTAGCCC    300
   ACAAAAGAAT GAGGACAGTG ACGAACTATT TTCTGGTGAA CCTGGCCTTC GCGGAGGCCT    360
   CCATGGCTGC ATTCAATACA GTGGTGAACT TCACCTATGC TGTCCACAAC GAATGGTACT    420
   ACGGCCTGTT CTACTGCAAG TTCCACAACT TCTTCCCCAT CGCCGCTGTC TTCGCCAGTA    480
   TCTACTCCAT GACGGCTGTG GCCTTTGATA GGTACATGGC CATCATACAT CCCCTCCAGC    540
   CCCGGCTGTC AGCCACAGCC ACCAAAGTGG TCATCTGTGT CATCTGGGTC CTGGCTCTCC    600
   TGCTGGCCTT CCCCCAGGGC TACTACTCAA CCACAGAGAC CATGCCCAGC AGAGTCGTGT    660
   GCATGATCGA ATGGCCAGAG CATCCGAACA AGATTTATGA GAAAGTGTAC CACATCTGTG    720
   TGACTGTGCT GATCTACTTC CTCCCCCTGC TGGTGATTGG CTATGCATAC ACCGTAGTGG    780
   GAATCACACT ATGGGCCAGT GAGATCCCCG GGGACTCCTC TGACCGCTAC CACGAGCAAG    840
   TCTCTGCCAA GCGCAAGGTG GTCAAAATGA TGATTGTCGT GGTGTGCACC TTCGCCATCT    900
   GCTGGCTGCC CTTCCACATC TTCTTCCTCC TGCCCTACAT CAACCCAGAT CTCTACCTGA    960
   AGAAGTTTAT CCAGCAGGTC TACCTGGCCA TCATGTGGCT GGCCATGAGC TCCACCATGT   1020
   ACAACCCCAT CATCTACTGC TGCCTCAATG ACAGGTTCCG TCTGGGCTTC AAGCATGCCT   1080
   TCCGGTGCTG CCCCTTCATC AGCGCCGGCG ACTATGAGGG GCTGGAAATG AAATCCACCC   1140
   GGTATCTCCA GACCCAGGGC AGTGTGTACA AAGTCAGCCG CCTGGAGACC ACCATCTCCA   1200
   CAGTGGTGGG GGCCCACGAG GAGGAGCCAG AGGACGGCCC CAAGGCCACA CCCTCGTCCC   1260
   TGGACCTGAC CTCCAACTGC TCTTCACGAA GTGACTCCAA GACCATGACA GAGAGCTTCA   1320
   GCTTCTCCTC CAATGTGCTC TCCTAG                                        1346

### (2) INFORMATION FOR SEQ ID NO:29:

(i) SEQUENCE CHARACTERISTICS:
   (A) LENGTH: 407 amino acids
   (B) TYPE: amino acid
   (C) STRANDEDNESS: single
   (D) TOPOLOGY: linear
(ii) MOLECULE TYPE:
(xi) SEQUENCE DESCRIPTION: SEQ ID NO:29:
   Met Asp Asn Val Leu Pro Val Asp Ser Asp Leu Ser Pro Asn Ile Ser
   1               5                   10                  15
   Thr Asn Thr Ser Glu Pro Asn Gln Phe Val Gln Pro Ala Trp Gln Ile
               20                  25                  30
   Val Leu Trp Ala Ala Ala Tyr Thr Val Ile Val Val Thr Ser Val Val
           35                  40                  45
   Gly Asn Val Val Val Met Trp Ile Ile Leu Ala His Lys Arg Met Arg
       50                  55                  60
   Thr Val Thr Asn Tyr Phe Leu Val Asn Leu Ala Phe Ala Glu Ala Ser
   65                  70                  75                  80
   Met Ala Ala Phe Asn Thr Val Val Asn Phe Thr Tyr Ala Val His Asn
                   85                  90                  95
   Glu Trp Tyr Tyr Gly Leu Phe Tyr Cys Lys Phe His Asn Phe Phe Pro
               100                 105                 110
   Ile Ala Ala Val Phe Ala Ser Ile Tyr Ser Met Thr Ala Val Ala Phe
           115                 120                 125
   Asp Arg Tyr Met Ala Ile Ile His Pro Leu Gln Pro Arg Leu Ser Ala
       130                 135                 140
   Thr Ala Thr Lys Val Val Ile Cys Val Ile Trp Val Leu Ala Leu Leu
   145                 150                 155                 160
   Leu Ala Phe Pro Gln Gly Tyr Tyr Ser Thr Thr Glu Thr Met Pro Ser
                   165                 170                 175
   Arg Val Val Cys Met Ile Glu Trp Pro Glu His Pro Asn Lys Ile Tyr
               180                 185                 190
   Glu Lys Val Tyr His Ile Cys Val Thr Val Leu Ile Tyr Phe Leu Pro
           195                 200                 205
   Leu Leu Val Ile Gly Tyr Ala Tyr Thr Val Val Gly Ile Thr Leu Trp
       210                 215                 220
   Ala Ser Glu Ile Pro Gly Asp Ser Ser Asp Arg Tyr His Glu Gln Val
   225                 230                 235                 240
   Ser Ala Lys Arg Lys Val Val Lys Met Met Ile Val Val Val Cys Thr
                   245                 250                 255
   Phe Ala Ile Cys Trp Leu Pro Phe His Ile Phe Phe Leu Leu Pro Tyr
               260                 265                 270
   Ile Asn Pro Asp Leu Tyr Leu Lys Lys Phe Ile Gln Gln Val Tyr Leu
           275                 280                 285
   Ala Ile Met Trp Leu Ala Met Ser Ser Thr Met Tyr Asn Pro Ile Ile
       290                 295                 300
   Tyr Cys Cys Leu Asn Asp Arg Phe Arg Leu Gly Phe Lys His Ala Phe
   305                 310                 315                 320
   Arg Cys Cys Pro Phe Ile Ser Ala Gly Asp Tyr Glu Gly Leu Glu Met
                   325                 330                 335
   Lys Ser Thr Arg Tyr Leu Gln Thr Gln Gly Ser Val Tyr Lys Val Ser
               340                 345                 350
   Arg Leu Glu Thr Thr Ile Ser Thr Val Val Gly Ala His Glu Glu Glu
           355                 360                 365
   Pro Glu Asp Gly Pro Lys Ala Thr Pro Ser Ser Leu Asp Leu Thr Ser
       370                 375                 380
   Asn Cys Ser Ser Arg Ser Asp Ser Lys Thr Met Thr Glu Ser Phe Ser
   385                 390                 395                 400
   Phe Ser Ser Asn Val Leu Ser
                   405

### (2) INFORMATION FOR SEQ ID NO:30:

(i) SEQUENCE CHARACTERISTICS:
   (A) LENGTH: 1679 base pairs
   (B) TYPE: nucleic acid
   (C) STRANDEDNESS: single
   (D) TOPOLOGY: linear
(ii) MOLECULE TYPE:
(xi) SEQUENCE DESCRIPTION: SEQ ID NO:30:
   GAAAAAGCCT TCCACCCTCC TGTCTGGCTT TAGAAGGACC CTGAGCCCCA GGCGCCACGA 60
   CAGGACTCTG CTGCAGAGGG GGGTTGTGTA CAGATAGTAG GGCTTTACCG CCTAGCTTCG 120
   AAATGGATAA CGTCCTCCCG GTGGACTCAG ACCTCTCCCC AAACATCTCC ACTAACACCT 180
   CGGAACCCAA TCAGTTCGTG CAACCAGCCT GGCAAATTGT CCTTTGGGCA GCTGCCTACA 240
   CGGTCATTGT GGTGACCTCT GTGGTGGGCA ACGTGGTAGT GATGTGGATC ATCTTAGCCC 300
   ACAAAAGAAT GAGGACAGTG ACGAACTATT TTCTGGTGAA CCTGGCCTTC GCGGAGGCCT 360
   CCATGGCTGC ATTCAATACA GTGGTGAACT TCACCTATGC TGTCCACAAC GAATGGTACT 420
   ACGGCCTGTT CTACTGCAAG TTCCACAACT TCTTCCCCAT CGCCGCTGTC TTCGCCAGTA 480
   TCTACTCCAT GACGGCTGTG GCCTTTGATA GGTACATGGC CATCATACAT CCCCTCCAGC 540
   CCCGGCTGTC AGCCACAGCC ACCAAAGTGG TCATCTGTGT CATCTGGGTC CTGGCTCTCC 600
   TGCTGGCCTT CCCCCAGGGC TACTACTCAA CCACAGAGAC CATGCCCAGC AGAGTCGTGT 660
   GCATGATCGA ATGGCCAGAG CATCCGAACA AGATTTATGA GAAAGTGTAC CACATCTGTG 720
   TGACTGTGCT GATCTACTTC CTCCCCCTGC TGGTGATTGG CTATGCATAC ACCGTAGTGG 780
   GAATCACACT ATGGGCCAGT GAGATCCCCG GGGACTCCTC TGACCGCTAC CACGAGCAAG 840
   TCTCTGCCAA GCGCAAGGTG GTCAAAATGA TGATTGTCGT GGTGTGCACC TTCGCCATCT 900
   GCTGGCTGCC CTTCCACATC TTCTTCCTCC TGCCCTACAT CAACCCAGAT CTCTACCTGA 960
   AGAAGTTTAT CCAGCAGGTC TACCTGGCCA TCATGTGGCT GGCCATGAGC TCCACCATGT 1020
   ACAACCCCAT CATCTACTGC TGCCTCAATG ACAGGTTCCG TCTGGGCTTC AAGCATGCCT 1080
   TCCGGTGCTG CCCCTTCATC AGCGCCGGCG ACTATGAGGG GCTGGAAATG AAATCCACCC 1140
   GGTATCTCCA GACCCAGGGC AGTGTGTACA AAGTCAGCCG CCTGGAGACC ACCATCTCCA 1200
   CAGTGGTGGG GGCCCACGAG GAGGAGCCAG AGGACGGCCC CAAGGCCACA CCCTCGTCCC 1260
   TGGACCTGAC CTCCAACTGC TCTTCACGAA GTGACTCCAA GACCATGACA GAGAGCTTCA 1320
   GCTTCTCCTC CAATGTGCTC TCCTAGGCCA CAGGGCCTTT GGCAGGTGCA GCCCCCACTG 1380
   CCTTTGACCT GCCTCCCTTC ATGCATGGAA ATTCCCTTCA TCTGGAACCA TCAGAAACAC 1440
   CCTCACACTG GGACTTGCAA AAAGGGTCAG TATGGGTTAG GGAAAACATT CCATCCTTGA 1500
   GTCAAAAAAT CTCAATTCTT CCCTATCTTT GCCACCCTCA TGCTGTGTGA CTCAAACCAA 1560
   ATCACTGAAC TTTGCTGAGC CTGTAAAATA AAAGGTCGGA CCAGCTTTTC CCAAAAGCCC 1620
   ATTCATTCCA TTCTGGAAGT GACTTTGGCT GCATGCGAGT GCTCATTTCA GGATGAATT 1679